# EUROPEAN PATENT APPLICATION

(11) **EP 4 813 450 A1**
(43) Date of publication of application: **30.09.2026**
(21) Application number: 26166093.0
(22) Date of filing: 19.03.2026
(51) Int. Cl.: A61N 5/10

(54) **RADIATION TREATMENT PLAN OPTIMIZATION METHOD AND APPARATUS**

(30) Priority: 27.03.2025 US 202519092178
(71) Applicant: Siemens Healthineers International AG, 6312 Steinhausen (CH)
(72) Inventor: RUOKOKOSKI, Emmi, 00100 Helsinki (FI); KUUSELA, Esa, 00100 Helsinki (FI); BASIRI, Shahab, 00100 Helsinki (FI); PIETILA, Ville, 00100 Helsinki (FI); MYLLYKOSKI, Mirko, 00100 Helsinki (FI); RUSANEN, Marko, 00100 Helsinki (FI)
(74) Representative: Mathisen & Macara LLP

(57) **Abstract**

To facilitate optimizing a radiation treatment plan for a particular patient using a particular radiation treatment machine having a multi-leaf collimator, a control circuit 101 can access a treatment duration parameter 201 and then optimize 203 the radiation treatment plan using a cost function having a term that is dependent on the treatment duration parameter to then provide an optimized radiation treatment plan having an optimized delivery time.

## Description

### TECHNICAL FIELD

These teachings relate generally to treating a patient's planning target volume with energy pursuant to an energy-based treatment plan and more particularly to optimizing an energy-based treatment plan.

### BACKGROUND

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-machine parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

A typical radiation treatment plan is created using an optimization algorithm that tries to minimize a cost function that comprises one or more user-defined optimization objectives. Typically, these objectives are dose objectives aiming to maximize target dose coverage and homogeneity while minimizing the dose to organs-at-risk.

### BRIEF DESCRIPTION OF DRAWINGS

According to a first aspect of the invention, there is provided a method as defined in claim 1. Optional features are specified in the dependent claims.

According to a second aspect of the invention, there is provided apparatus as defined in claim 10. Optional features are specified in the dependent claims. The apparatus may be configured to administer therapeutic radiation to the particular patient using the radiation treatment machine and the optimized radiation treatment plan.

According to a third aspect of the invention, there is provided a non-transitory computer-readable medium, as defined in claim 14. The non-transitory computer-readable medium instructions, when executed on a processor, may control administering therapeutic radiation to the particular patient using the radiation treatment machine and the optimized radiation treatment plan.

The radiation treatment machine may have a multi-leaf collimator.

### BRIEF DESCRIPTION OF DRAWINGS

Various needs are at least partially met through provision of the radiation treatment plan optimization method and apparatus described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings;
FIG. 3 comprises a graph as configured in accordance with various embodiments of these teachings;
FIG. 4 comprises a graph as configured in accordance with various embodiments of these teachings; and
FIG. 5 comprises a graph as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### DETAILED DESCRIPTION

Generally speaking, these various embodiments can serve to facilitate optimizing a radiation treatment plan for a particular patient using a particular radiation treatment machine having a multi-leaf collimator. In these regards, a control circuit can access a treatment duration parameter and then optimize the radiation treatment plan using a cost function having a term that is dependent on the treatment duration parameter to then provide an optimized radiation treatment plan having an optimized delivery time.

By one approach, the treatment duration parameter can correspond to a time-based physiological limit for the particular patient. As one illustrative example in these regards, that time-based physiological limit can correspond to a duration of time during which at least a targeted portion of the particular patient can remain substantially immobile. By one approach, the treatment duration parameter can comprise a user-selected treatment duration parameter.

By one approach, optimizing the radiation treatment plan can comprise using a soft maximum as a smooth approximation of a maximum function to improve numerical stability of the optimizing. By one approach, in lieu of the foregoing or in combination therewith, optimizing the radiation treatment plan can comprise representing radiation treatment machine axes in the cost function, which radiation treatment machine axes (such as, but not limited to, predetermined radiation source positions) are not to be optimized.

By one approach, the aforementioned cost function can further include a dosimetric component to be optimized. And by another approach, in lieu of the foregoing or in combination therewith, the cost function can comprise a monitor unit time-of-delivery component.

By one optional approach, these teachings will accommodate administering therapeutic radiation to the particular patient using the radiation treatment machine and the optimized radiation treatment plan.

By one approach, these teachings can comprise a non-transitory computer-readable medium having a computer program that itself comprises instructions that, when the computer program is executed by a computer (such as a processor), carries out any one or more of the steps, actions, and/or functions described herein, including causing the computer to carry out accessing a treatment duration parameter and then optimizing a radiation treatment plan using a cost function having a term that is dependent on the treatment duration parameter to then provide an optimized radiation treatment plan having an optimized delivery time.

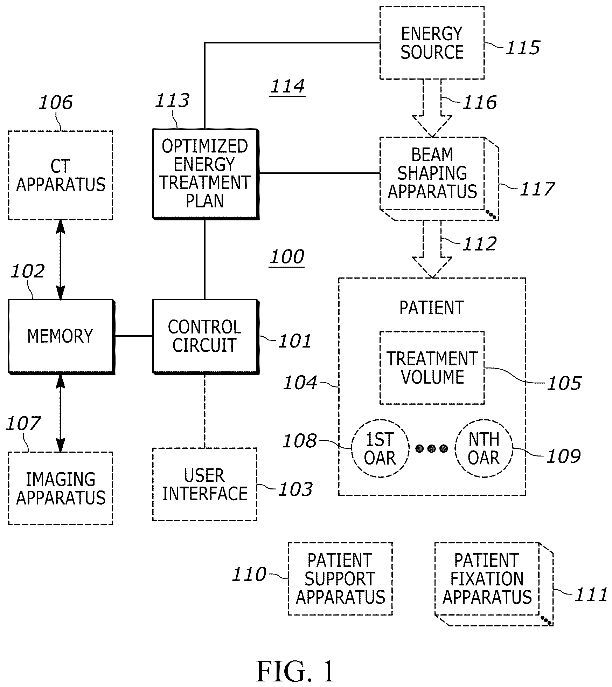

Various benefits may become evident upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware machine (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware machine (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory machines that collectively comprise the "memory" of this apparatus 100.

In addition to information such as optimization information for a particular patient and information regarding a particular radiation treatment machine as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both non-volatile memory (such as read-only memory (ROM) as well as volatile memory (such as a dynamic random access memory (DRAM).)

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless machines, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-machine parameters during each of a plurality of sequential exposure fields. In this case the energy-based treatment plan is generated through an optimization process, examples of which are provided further herein.

By one approach the control circuit 101 can operably couple to an energy-based treatment machine 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment machines/apparatuses. In a typical application setting the energy-based treatment machine 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment machine 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

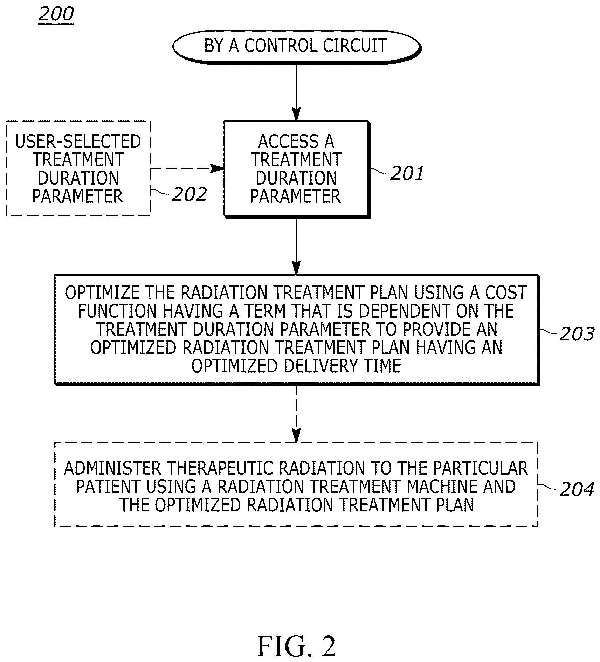

Referring now to FIG. 2, a process 200 that can be carried out, for example, in conjunction with the above-described application setting (and more particularly via the aforementioned control circuit 101) will be described. Generally speaking, this process 200 serves to facilitate generating an optimized radiation treatment plan 113 to thereby facilitate optionally treating a particular patient with therapeutic radiation using a particular radiation treatment machine per that optimized radiation treatment plan.

At block 201, the control circuit 101 accesses a treatment duration parameter (for example, by retrieving that parameter from the aforementioned memory 102). By one approach, the treatment duration parameter corresponds to a time-based physiological limit for the particular patient. For example, the time-based physiological limit can correspond to a duration of time during which at least a targeted portion of the particular patient can remain substantially immobile. An illustrative example in these regards is a duration of time during which the particular patient can reliably hold their breath in order to halt breathing-related movement of their body.

By one approach, and as illustrated by optional block 202, the treatment duration parameter can comprise a user-selected treatment duration parameter. Such a selection may be entered, for example, via the above-described user interface 103.

By one approach, a value for this parameter can be empirically determined by having the particular patient hold their breath a given number of times and timing the duration of each such effort. The average duration might be used as the treatment duration parameter, or the shortest duration of those various efforts might be used as the treatment duration parameter, or some other approach may be utilized as desired.

At block 203, the control circuit 101 optimizes the radiation treatment plan using a cost function having a term that is dependent on the treatment duration parameter to provide an optimized radiation treatment plan having an optimized delivery time. A cost function, also known as an objective function or a loss function in certain contexts, is a mathematical formulation used in optimization algorithms to evaluate how well a particular solution or set of parameters fulfills the desired outcome. The cost function takes the algorithm's input parameters and computes a numerical cost associated with those parameters, effectively quantifying the error or distance from the optimal solution. In optimization problems, the goal is to find the set of parameters that minimize or maximize the cost function, depending on the specific problem at hand. Minimization is more common, where the objective is to find the parameters that result in the lowest possible cost, indicating the best or most efficient solution under the defined criteria.

As one illustrative example, that cost function can have a term that is dependent on a time-based physiological limit for the particular patient, such as a duration of time during which the particular patient can reliably hold their breath. Such optimization can presume, by one approach, only a single such breath-holding episode. By another approach, such optimization can presume a plurality of breath-holding episodes separated by some intervals of time (and perhaps taken on a control point-by-control point basis).

By one approach, the cost function can further include a dosimetric component to be optimized (such as a desired dosing of a patient's target volume and/or a maximum dosing limit for a non-targeted organ/tissue).

In lieu of the foregoing or in combination therewith, the cost function can also optionally comprise a monitor unit time-of-delivery component. A monitor unit (MU) is a standardized measure of the dose output from a radiation treatment machine, such as a linear accelerator, and can be used to calibrate the delivery of the prescribed radiation dose to a patient's target tissue. The monitor unit is directly related to the amount of charge produced by ionization in a reference volume of air under specified conditions, providing a quantifiable relationship between the machine settings and the actual dose delivered. During treatment planning, the number of monitor units can be calculated to ensure that the intended dose is accurately administered to the patient, taking into account factors such as machine calibration, beam energy, treatment geometry, and patient-specific characteristics.

By one approach, optimizing the radiation treatment plan can comprise using a soft maximum as a smooth approximation of a maximum function to improve numerical stability of the optimizing process. The applicant has determined that using a hard maximum function in the optimization process, which strictly enforces a sharp cutoff at a certain dose level, can lead to numerical instability. This instability can arise because the optimization algorithm might struggle to find a solution that precisely meets the hard constraints, leading to erratic or non-convergent behavior. A soft maximum can instead be introduced as a smooth approximation of the maximum function. This soft maximum allows for a more flexible approach by penalizing doses that exceed a certain threshold, rather than outright prohibiting them. This penalty can be incorporated into the optimization objective function as a term that becomes progressively larger as the dose goes beyond the desired limit. The applicant has determined that use of a soft maximum can help to smooth out the optimization landscape, making it easier for the algorithm to find a stable and optimal solution.

In lieu of the foregoing or in combination there with, these teachings will accommodate the cost function also comprising a monitor unit time-of-delivery component.

So configured, the optimization process can optimize, at least in part, with respect to assuring adequate dosing of the target volume (and appropriate protection, where desired, of organs-at-risk) notwithstanding a need to accommodate a patient's breathing behavior.

As illustrated by optional block 204, these teachings will also accommodate administering therapeutic radiation to the particular patient using the radiation treatment machine and the aforementioned optimized radiation treatment plan. Administering the radiation pursuant to such a plan can include, for at least portions of the treatment, causing the radiation beam to be off at times when the particular patient is breathing and to permit the radiation beam to be on at times when the particular patient is not breathing.

Further details that comport with these teachings will now be presented. It will be understood that the specific details of these examples are intended to serve an illustrative purpose and are not intended to suggest any particular limitations with respect to these teachings.

In time-critical treatments, it can be beneficial to be able to control the treatment delivery time. Examples of such treatments are breath-hold lung treatments and palliative treatments. In lung breath-hold treatments the planner often considers the patient's capability to hold their breath and will seek to plan the treatment in such a way that it can be delivered in the breath-hold time in an accurate way. If the treatment delivery is interrupted in a high dose rate part of the delivery trajectory (for example, upon detecting breathing-based movement of the patient), the accuracy of the delivered dose may be compromised. In palliative treatments the patient may be in considerable pain which limits their ability to lay still during the treatment.

In many prior art approaches, the delivery time is controlled indirectly by limiting the amount of monitor units an a monitor units objective. This approach, however, controls only one of the many axes that can contribute to the final delivery time. Furthermore, the amount of monitor units gives an indication of the delivery time but the actual time it takes to deliver the monitor units depends on how the monitor units are distributed along the delivery trajectory as well as various machine parameters (such as maximum monitor unit/arc degree, maximum dose rate, and so forth).

The following illustrative example comprises a specific implementation for using delivery time as an optimization objective to address a situation such as those described above. When delivery time is passed to the treatment plan optimizer as an objective, a treatment-time dependent term is added into the cost function for leaf sequencing. By one approach, this term can be an arbitrary function of treatment time *Cₜᵢₘₑ* (*t*):R→R.

First, this example will address the time component of the cost function.

The cost function in the leaf sequencing can be as follows: ${C}_{tot} \left(X\right) = {C}_{dos} \left({F}_{target},F\left(X\right)\right) + {C}_{time} \left(t\left(X\right)\right) ,$ where the total cost *Cₜₒₜ* comprises the dosimetric component *C_{dos}* and the treatment-time dependent term *Cₜᵢₘₑ. X* is the total position vector containing *xᵢⱼ* for every axis *j*, at every control point *i, F(X)* is the fluence as a function of axis positions, and *t(X)* is the total delivery time. The total delivery time can be approximated as a sum of individual control point interval times *t(X)* = Σ*ᵢ tᵢ.* The interval time, in turn, is the maximum of individual axis times ${t}_{i} = {max}_{j} {t}_{ji}$*,* where *tⱼᵢ* is the minimum time that axis *j* takes in interval *i.* For axis *j* this time can be calculated as ${t}_{ji} = Δ {x}_{ji} / {v}_{j}^{max}$*,* where Δ*xⱼᵢ = xⱼᵢ - x*_{*ji-*1} (the amount of change in axis *j* in control point *i),* and ${v}_{j}^{max}$ is the maximum velocity of axis *j*. Similarly, the slowest axis at interval *i* with index ${j}_{i}^{∗}$ is defined as ${j}_{i}^{∗} = {argmax}_{j} {t}_{ji} .$ *.*

Often an efficient optimization process requires calculation of the gradient of the cost function at the neighborhood of a known solution. For the aforementioned cost function the gradient relative to a given leaf position (or any optimized degree of freedom) can be calculated as: ${∇}_{{x}_{ji}} {C}_{tot} \left(X\right) = {∇}_{{x}_{ji}} {C}_{dos} \left({F}_{target},F\left(X\right)\right) + {∇}_{{x}_{ji}} {C}_{time} \left(t\left(X\right)\right)$

The gradient of the treatment time term can be expressed as ${∇}_{{x}_{ji}} {C}_{time} = {\partial }_{t} {C}_{time} \left(t\right) ⋅ {∇}_{{x}_{ji}} t \left(X\right)$ where the first term can be calculated based on the high-level utility of a certain treatment time, and the latter part can take into account how a certain axis position in a certain control point is affecting the total time.

Viewed one way, only the slowest axis affects that total time. ${∇}_{{x}_{ji}} t \left(X\right) = {\partial }_{{x}_{ji}} \left({t}_{i}+{t}_{i + 1}\right) = \left({δ}_{{jj}_{i}^{∗}}-{δ}_{{jj}_{i + 1}^{∗}}\right) / {v}_{j}^{max}$ where *δ_{jj'}* is a symbol for the Kronecker delta.

Next, this example will address the gradient of the time component with a soft maximum.

To improve the numerical stability of the optimizer, in this example the max in the formula of ${t}_{i} = {max}_{j} {t}_{ji}$ is replaced with a soft max function or a normalized exponential function. The soft max function can be considered as a generalization of a standard max function. Considering the generalized weighted average expressed as *t*_{{*w*}} *=* ∑*ⱼwⱼᵢtⱼᵢ*/Σ*ⱼwⱼᵢ,* a classic max function can be obtained by selecting ${w}_{ji} = \left\{\begin{matrix}1 , & {t}_{ji} \geq {t}_{j ′ i} ∀ j ′ \\ 0 , & otherwise\end{matrix}\right. .$

**For** softmax, one can replace *wⱼᵢ* with *qⱼᵢ* = exp(β*tⱼᵢ*): ${t}_{i} = smax \left(\left\{t\right\}\right) = \frac{{\sum }_{j} {q}_{ji} {t}_{ji}}{{\sum }_{j} {q}_{ji}} = \frac{{\sum }_{j} exp \left({βt}_{ji}\right) {t}_{ji}}{{\sum }_{j} exp \left({βt}_{ji}\right)} , {t}_{ji} = \frac{{x}_{ji} - {x}_{ji - 1}}{{v}_{j}^{max}}$

These teachings will accommodate other approaches for replacing the max with a softer function if desired. By one approach, for example, one could utilize In(∑*ⱼ* exp(*βtⱼᵢ*))*.*

The gradient can then be formatted as: ${∇}_{{x}_{ji}} t \left(X\right) = \frac{\left({Ξ}_{ij}-{Ξ}_{ji + 1}\right)}{{v}_{j}^{max}}$ where ${Ξ}_{ji} = \left(\left({βt}_{ji}+1\right){Π}_{i}-β{\sum }_{j ′} {q}_{j ′ i} {t}_{j ′ i}\right) {q}_{ji} {Π}_{i}^{- 2}$,
and Πᵢ = Σ*ⱼ qⱼᵢ.*

Note that *β* can be (and should be if dimensions of *xᵢ's* are different) different for each *i* (that is, one can replace *β* → *βᵢ*).

So configured, applying the soft maximum approach can accord a higher cost to the axes that are causing the machine to most significantly slow down.

By one approach, these teachings can be viewed as dividing all of the treatment machine degrees-of-freedom into three categories: (1) non-optimized machine axes that have been defined prior to optimization (for example, gantry angles are typically set for each control point at the beginning of the optimization or prior to calling the optimizer and they are not necessarily changed during optimization; (2) freely-optimized parameters as described above (note that their amount of motion is not limited directly but only via the treatment time constraints; and (3) restrictively optimized parameters where the optimizer is allowed to change the parameter so that the parameter will not slow down the treatment (regardless of the total treatment time). Note that only the first two above-designated categories appear in the time cost function term since the variables in the third category are not affecting the treatment time.

As a specific example, consider a scenario where the leaf positions are considered to be treated according to the third category specified above and the gantry positions are to be treated according to the first category specified above. The control point spacing in the gantry axis can be set so that the gantry motion always requires time *t_{g}*. This time is the time it takes to move the gantry at full speed between two consecutive control points.

The only optimizable variable that corresponds to the second category described above, then, are the delivered monitor units during the control point interval *muᵢ.* Thus, there are only two values in the softmax calculation. In this example, these teachings will focus on the time cost gradient for the monitor units. The maximum velocity for *muᵢ* is the maximum dose rate *dᵣₐₜₑ.*

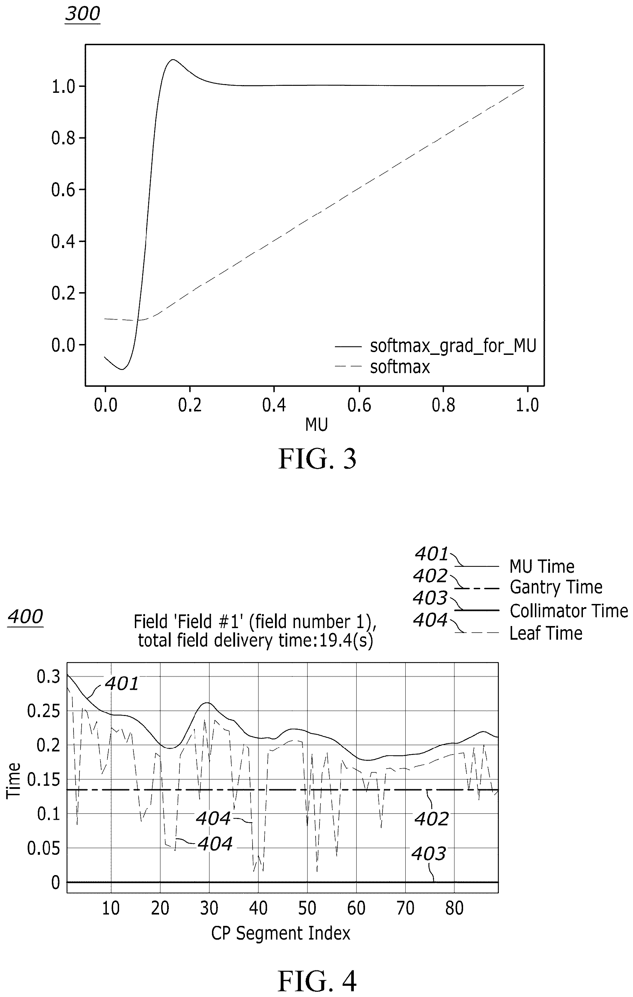

A graph 300 presented at FIG. 3 presents the time cost as ${C}_{t} \left({\sum }_{i} {smax}_{j} \left(Δ{x}_{ji}/{v}_{j}^{max}\right)\right) = {C}_{t} \left({\sum }_{i} {smax}_{j} \left({mu}_{i}/{d}_{rate},{t}_{g}\right)\right)$

Now consider an example using the soft max and the gradient with *t_{g}* = 0.1 seconds. In particular, and referring to the graph 400 shown in FIG. 4, the soft max time cost term can be applied to a real hypofractionated lung case where *t_{g}* ≈ 0.134 seconds. First, in this example, a lung case is optimized without a delivery time objective. In this graph 400, the x-axis corresponds to the control point index and the *y-*axis represents the time in seconds. Of particular note are the line 401 that presents the time it takes to deliver the monitor units in this control point interval and the line 402 that corresponds to the gantry time. (Line 403 corresponds to the collimator time and line 404 corresponds to the leaf time.)

It may be noted that the total delivery time in this example is 19.4 seconds.

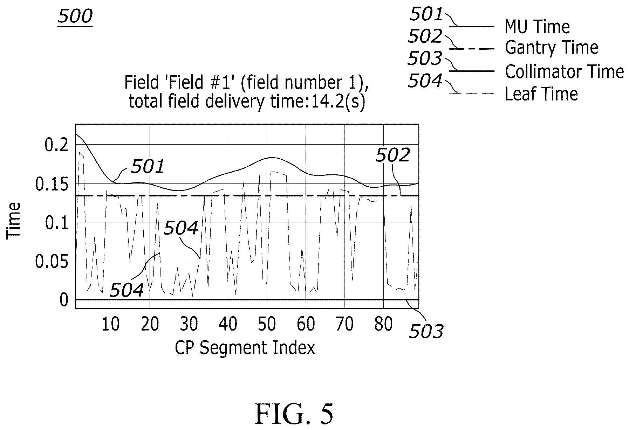

In this next example, the lung case is instead optimized in accordance with these teachings with a delivery time objective of 14 seconds (to accommodate, for example, a patient who cannot reliably hold their breath any longer than 15 seconds). Corresponding optimization results are shown in FIG. 5. Compare and contrast the monitor unit time line 501, the gantry time line 502, the collimator time line 503, and the leaf time line 504 with the corresponding results in the previous example (as shown in FIG. 4), and note in particular that the total field delivery time is now 14.2 seconds, a significant reduction from the 19.4 seconds achieved by the previous example.

These teachings allow a user/planner to explicitly define a desired delivery time (or desired upper limit for the delivery time), with time estimations being based on up to all axes of the treatment machine if desired.

Further aspects of these teachings are provided by the subject matter of the following clauses (where it will be understood that any of these clauses can be combined with any one of more of the other clauses as desired).

Clause 1. A method of optimizing a radiation treatment plan for a particular patient using a particular radiation treatment machine having a multi-leaf collimator, the method comprising: by a control circuit: accessing a treatment duration parameter; optimizing the radiation treatment plan using a cost function having a term that is dependent on the treatment duration parameter to provide an optimized radiation treatment plan having an optimized delivery time.

Clause 2. The method of clause 1 wherein the treatment duration parameter comprises a user-selected treatment duration parameter.

Clause 3. The method of clause 1 wherein the treatment duration parameter corresponds to a time-based physiological limit for the particular patient.

Clause 4. The method of clause 3 wherein the time-based physiological limit corresponds to a duration of time during which at least a targeted portion of the particular patient can remain substantially immobile.

Clause 5. The method of clause 1 wherein the cost function further includes a dosimetric component to be optimized.

Clause 6. The method of clause 1 wherein optimizing the radiation treatment plan comprises using a soft maximum as a smooth approximation of a maximum function to improve numerical stability of the optimizing.

Clause 7. The method of clause 1 wherein optimizing the radiation treatment plan comprises representing radiation treatment machine axes in the cost function, which radiation treatment machine axes are not to be optimized.

Clause 8. The method of clause 7 wherein the radiation treatment machine axes comprise predetermined radiation source positions.

Clause 9. The method of clause 1 wherein the cost function comprises a monitor unit time-of-delivery component.

Clause 10. The method of clause 1 further comprising: administering therapeutic radiation to the particular patient using the radiation treatment machine and the optimized radiation treatment plan.

Clause 11. An apparatus for optimizing a radiation treatment plan for a particular patient using a particular radiation treatment machine having a multi-leaf collimator, the apparatus comprising: a control circuit configured to: access a treatment duration parameter; optimize the radiation treatment plan using a cost function having a term that is dependent on the treatment duration parameter to provide an optimized radiation treatment plan having an optimized delivery time.

Clause 12. The apparatus of clause 11 wherein the treatment duration parameter comprises a user-selected treatment duration parameter.

Clause 13. The apparatus of clause 11 wherein the treatment duration parameter corresponds to a time-based physiological limit for the particular patient.

Clause 14. The apparatus of clause 13 wherein the time-based physiological limit corresponds to a duration of time during which at least a targeted portion of the particular patient can remain substantially immobile.

Clause 15. The apparatus of clause 11 wherein the cost function further includes a dosimetric component to be optimized.

Clause 16. The apparatus of clause 11 wherein the control circuit is configured to optimize the radiation treatment plan by using a soft maximum as a smooth approximation of a maximum function to improve numerical stability of the optimizing.

Clause 17. The apparatus of clause 11 wherein the control circuit is configured to optimize the radiation treatment plan by representing radiation treatment machine axes in the cost function, which radiation treatment machine axes are not to be optimized.

Clause 18. The apparatus of clause 17 wherein the radiation treatment machine axes comprise predetermined radiation source positions.

Clause 19. The apparatus of clause 11 wherein the cost function comprises a monitor unit time-of-delivery component.

Clause 20. A non-transitory computer-readable medium for optimizing a radiation treatment plan for a particular patient using a particular radiation treatment machine having a multi-leaf collimator, the non-transitory computer-readable medium comprising instructions stored thereon, that when executed on a processor, perform the steps of: accessing a treatment duration parameter; optimizing the radiation treatment plan using a cost function having a term that is dependent on the treatment duration parameter to provide an optimized radiation treatment plan having an optimized delivery time.

Those skilled in the art will recognize that a wide variety of modifications, alterations, and combinations can be made with respect to the above-described embodiments without departing from the scope of the invention. As one example in these regards, these teachings can be employed in conjunction with mechanisms to detect a patient's actual breathing behavior during the treatment process such that the administration of the radiation treatment plan may be modified in real time to accommodate that breathing behavior. Accordingly, such modifications, alterations, and combinations are to be viewed as being within the ambit of the inventive concept.

## Claims

1. A method of optimizing a radiation treatment plan for a particular patient using a particular radiation treatment machine, the method comprising:
by a control circuit:
accessing a treatment duration parameter;
optimizing the radiation treatment plan using a cost function having a term that is dependent on the treatment duration parameter to provide an optimized radiation treatment plan having an optimized delivery time.

2. The method of claim 1 wherein the treatment duration parameter comprises a user-selected treatment duration parameter.

3. The method of claim 1 wherein the treatment duration parameter corresponds to a time-based physiological limit for the particular patient.

4. The method of claim 3 wherein the time-based physiological limit corresponds to a duration of time during which at least a targeted portion of the particular patient can remain substantially immobile.

5. The method of any one of claims 1 to 4 wherein the cost function further includes a dosimetric component to be optimized.

6. The method of any one of claims 1 to 5 wherein optimizing the radiation treatment plan comprises using a soft maximum as a smooth approximation of a maximum function to improve numerical stability of the optimizing.

7. The method of any one of claims 1 to 6 wherein optimizing the radiation treatment plan comprises representing radiation treatment machine axes in the cost function, which radiation treatment machine axes are not to be optimized.

8. The method of any one of claims 1 to 7 wherein the radiation treatment machine axes comprise predetermined radiation source positions.

9. The method of any one of claims 1 to 8 wherein the cost function comprises a monitor unit time-of-delivery component.

10. An apparatus for optimizing a radiation treatment plan for a particular patient using a particular radiation treatment machine, the apparatus comprising:
a control circuit configured to:
access a treatment duration parameter;
optimize the radiation treatment plan using a cost function having a term that is dependent on the treatment duration parameter to provide an optimized radiation treatment plan having an optimized delivery time.

11. The apparatus of claim 10 wherein the control circuit is configured to optimize the radiation treatment plan by using a soft maximum as a smooth approximation of a maximum function to improve numerical stability of the optimizing.

12. The apparatus of claim 10 or 11 wherein the control circuit is configured to optimize the radiation treatment plan by representing radiation treatment machine axes in the cost function, which radiation treatment machine axes are not to be optimized.

13. The apparatus of claim 12 configured to perform the method of any one of claims 2 to 5, 8 or 9.

14. A non-transitory computer-readable medium for optimizing a radiation treatment plan for a particular patient using a particular radiation treatment machine, the non-transitory computer-readable medium comprising instructions stored thereon, that when executed on a processor, perform the steps of:
accessing a treatment duration parameter;
optimizing the radiation treatment plan using a cost function having a term that is dependent on the treatment duration parameter to provide an optimized radiation treatment plan having an optimized delivery time;
wherein, optionally, the instructions, when executed on a processor, perform the method of any one of claims 1 to 9.

15. The method, apparatus or non-transitory computer-readable medium of any one of the preceding claims wherein the radiation treatment machine has a multi-leaf collimator.
